# EUROPEAN PATENT SPECIFICATION

(11) **EP 3 052 051 B1**
(45) Date of publication and mention of the grant of the patent: **12.06.2019**
(21) Application number: 14847120.4
(22) Date of filing: 29.09.2014
(51) Int. Cl.: A61F 2/06, A61L 29/16, A61L 29/08, A61L 33/00, A61L 33/08, A61L 29/06

(54) **STABILIZED ENZYME COMPOSITIONS**
STABILISIERTE ENZYMZUSAMMENSETZUNGEN
COMPOSITIONS D'ENZYMES STABILISÉES

(30) Priority: 30.09.2013 US 201314041947
(43) Date of publication of application: 10.08.2016
(73) Proprietor: Teleflex Medical Incorporated, Morrisville, North Carolina 27560 (US)
(72) Inventor: GUPTA, Nisha, Audobon, Pennsylvania 19403 (US)
(74) Representative: CMS Cameron McKenna Nabarro Olswang LLP
(86) International application number: PCT/US2014/057984
(87) International publication number: WO 2015/048619

(56) References cited:
- WO-A1-2013/130459
- WO-A2-2004/073504
- US-A1- 2010 215 711
- US-B1- 6 248 712
- CHIN ET AL.: 'Antimicrobial Activities of Ceragenins against Clinical Isolates of Resistant Staphylococcus aureus .' ANTIMICROBIAL AGENTS AND CHEMOTHERAPY vol. 51, no. 4, 01 April 2007, pages 1268 - 1273, XP055052269 DOI: 10.1128/AAC.01325-06

## Description

### FIELD OF THE INVENTION

The present invention generally relates to enzyme compositions. More particularly, the present invention pertains to stabilized enzyme compositions, medical devices having stabilized enzyme compositions, and method of production thereof.

### BACKGROUND OF THE INVENTION

Implantable medical devices such as tunneled catheters play a major role in general medicine. Within days of insertion, almost all central venous catheters are coated with a fibrin sheath, and within 30 days, most catheter-related thrombi arise. (See C Harter, HJ Salwender, A Bach, G Egerer, H Goldschmidt and AD Ho (2002) Catheter-related infection and thrombosis of the internal jugular vein in hematologic-oncologic patients undergoing chemotherapy: a prospective comparison of silver-coated and uncoated catheters. Cancer 94: 245-251.) Aside from reducing the function of the catheter, these catheter-related thrombi can cause postphlebitic syndrome in 15%-30% cases and pulmonary embolism in 11% of the cases. (See DJ Kuter (2004) Thrombotic Complications of Central Venous Catheters in Cancer Patients. The Oncologist 9: 207-216.)

Examples of immobilized fibrinolytic enzymes are disclosed in U.S. Patent Nos. 4,305,926, 4,273,873, 4,378,435, and 5,380,299. Unfortunately, the fibrinolytic activity from such devices, e.g. urokinase coated hemodialysis catheter from Unitika (Blood access UK-catheter, Unitika), is retained for less than two weeks. This duration is insufficient for long term or chronic devices. A variety of agents have been conventionally utilized to stabilize fibrinolytic enzymes in solution. Examples of agents utilized to stabilize fibrinolytic enzymes in solution are disclosed in the following foreign patents: Japanese Patent No. JP61238732A2; European Patent No. EP0200966B1; Canadian Patent No. CA2579458AA; European Patent No. EP0391400 A2; and Japanese Patent No. JP55034082A2. However, to date, these agents have not been successfully utilized in an implantable medical device.

Accordingly, it is desirable to provide an implantable medical device having extended thrombolytic properties that is capable of overcoming the disadvantages described herein at least to some extent.

US2010/215711 discloses a medical device including a base material having an immobilized fibrinolytic enzyme and dextran sulfate.

### SUMMARY OF THE INVENTION

The foregoing needs are met, to a great extent, by the present invention, which is defined by the claims.

An embodiment of the present invention pertains to a medical device according to claim 1. The medical device includes a base material having an immobilized extracellular matrix digestive enzyme covalently bound to the base material in the presence of dextran sulfate. The medical device is for vascular implantation in a patient. The dextran sulfate has a molecular weight that is 8 kilodaltons (kDa) to stabilize the enzyme when in contact with blood of the patient.

Another embodiment of the present invention relates to a medical device according to claim 6. The medical device includes a polyurethane base material, an amount of a blood clot digestive enzyme, an amount of an extracellular matrix digestive enzyme, a low molecular weight dextran sulfate, and an antimicrobial agent. The polyurethane base material is for vascular implantation in a patient. The amount of the blood clot digestive enzyme is sufficient to reduce the formation of device related blood clots. The amount of the extracellular matrix digestive enzyme is sufficient to reduce neo-initimal hyperplasia. The enzymes are immobilized by covalently binding the enzymes to the base material. The low molecular weight dextran sulfate is to stabilize the enzymes when in contact with blood of the patient. The low molecular weight dextran sulfate has a molecular weight of 8 kilodaltons. The antimicrobial agent is disposed in the polyurethane base material in an amount sufficient to reduce microbial growth.

Yet another embodiment of the present invention pertains to a medical device according to claim 7. The medical device includes a polyurethane base material, an amount of an enzyme, a low molecular weight dextran sulfate, and a chlorhexidine base or a pharmaceutically acceptable salt thereof. The polyurethane base material is for vascular implantation in a patient. The amount of the enzyme is sufficient to reduce neo-initimal hyperplasia. The enzyme is covalently immobilized in the base material. The low molecular weight dextran sulfate is to stabilize the enzyme when in contact with blood of the patient. The low molecular weight dextran sulfate has a molecular weight of 8 kilodaltons. The chlorhexidine base or a pharmaceutically acceptable salt thereof is disposed in the polyurethane base material in an amount sufficient to reduce microbial growth.

There has thus been outlined, rather broadly, certain embodiments of the invention in order that the detailed description thereof herein may be better understood, and in order that the present contribution to the art may be better appreciated. There are, of course, additional embodiments of the invention that will be described below and which will form the subject matter of the claims appended hereto.

In this respect, before explaining at least one embodiment of the invention in detail, it is to be understood that the invention is not limited in its application to the details of construction and to the arrangements of the components set forth in the following description or illustrated in the drawings. The invention is capable of embodiments in addition to those described and of being practiced and carried out in various ways. Also, it is to be understood that the phraseology and terminology employed herein, as well as the abstract, are for the purpose of description and should not be regarded as limiting.

As such, those skilled in the art will appreciate that the conception upon which this disclosure is based may readily be utilized as a basis for the designing of other structures, methods and systems for carrying out the several purposes of the present invention.

### BRIEF DESCRIPTION OF THE DRAWINGS

FIG. 1 is a graph showing fibrinolytic activity of a urokinase-type plasminogen activator (uPA) immobilized on polyurethane catheter segments following incubation times ranging from 0 to 28 days.
FIG. 2 is a graph showing urokinase activity of explanted catheter segments following incubation times of 0 and 14 days.
FIG. 3 is a graph showing fibrinolytic activity of uPA immobilized on polyurethane catheter segments and stabilized with low molecular weight dextran sulfate, human serum albumin, and the amino acid arginine following incubation times ranging from 0 to 28 days.
FIG. 4 is a graph showing fibrinolytic activity of uPA immobilized on chlorhexidine coated polyurethane catheter segments and stabilized with low molecular weight dextran sulfate, human serum albumin, and the amino acid arginine following incubation times ranging from 0 to 28 days.
FIG. 5 is a graph showing fibrinolytic activity of uPA immobilized on chlorhexidine coated TECOTHANE® catheter segments and stabilized with high molecular weight dextran sulfate and low molecular weight dextran sulfate following incubation times ranging from 0 to 9 days.
FIG. 6 is a graph showing fibrinolytic activity of uPA immobilized on chlorhexidine coated polyurethane catheter segments and stabilized with low molecular weight dextran sulfate, human serum albumin, and the amino acid arginine following incubation times ranging from 0 to 28 days.

### DETAILED DESCRIPTION

Embodiments of the invention provide an implantable medical device having extended enzymatic and optionally also antimicrobial properties. In various embodiments these extended enzymatic and optionally also antimicrobial properties may be achieved via the immobilization of various suitable enzymes and optionally antimicrobial agents on the surface of the implantable medical devices in the presence of dextran sulfate. Examples of suitable optional enzymes include blood clot digestive enzyme or fibrinolytic enzymes such as Urokinase (uPA), aminopeptidases, tissue plasminogen activator (tPA), streptokinase, and the like. According to the present invention the medical devices include extracellular matrix digestive enzyme including one or more of a protease; an aminopeptidase; a collagenase; a matrix metalloproteinase; a metalloendopeptidase; a serine endopeptidase; a hydrolase; a glucuronidase; a hyaluronoglucosaminidase; a heparanase; a cathepsin; a hyaluronidase; and a matriptase. Examples of suitable antimicrobial agents include; Chlorhexidine base, Alexidine, other guanides including polyhexamethylene biguanide (PHMB), cationic selective antimicrobials (CSAs), Ceragenins, and the like. Two molecular weight ranges of dextran sulfate are worth distinguishing. The first molecular weight range is generally referred to as high molecular weight dextran sulfate ("HMW-DexS") which has a molecular weight of greater than 40,000 dalton (Da) to about 500,000 Da or more. HMW-DexS has previously reported to inhibit inflammatory cell invasion around an implant. The second molecular weight range is generally referred to as low molecular weight dextran sulfate ("LMW-DexS") which has a molecular weight of less that about 40,000 Da to about 500 Da. The beneficial use of LMW-DexS on an implant has not been previously reported. It is therefore surprising and unexpected that LMW-DexS stabilizes and increases the duration of bioactivity of immobilized fibrinolytic enzymes and/or releasable antimicrobial agent(s) on the surface of a medical device that interfaces with blood. As described herein, according to the present invention use is made of LMW-DexS having a molecular weight of 8,000 Da.

In addition, it is within the purview of this and other embodiments of the invention that other suitable agents may be incorporated into the bulk material. Examples of suitable agents includes other antimicrobial agents, antibiotics, antiseptics, chemotherapeutics, antimicrobial peptides, mimetics, antithrombogenic, fibrinolytic, anticoagulants, anti-inflammatory, anti-pain, antinausea, vasodilators, antiproliferatives, antifibrotics, growth factors, cytokines, antibodies, peptide and peptide mimetics, nucleic acids, and/or the like.

Medical devices suitable for use with various embodiments of the invention may include catheters, tubes, sutures, non-woven, meshes, drains, shunts, stents, foams etc. Other devices suitable for use with embodiments of the invention include those that may interface with blood, blood products, and/or fibrinogenic fluids, tissues, and/or products.

Forms of chlorhexidine suitable for use with embodiments of the invention include chlorhexidine base, pharmaceutically acceptable chlorhexidine salts such as, for example, diacetate, laurate (dodecanoate), palmitate (hexadecanoate), myristate (tetradecanoate), stearate (octadecanoate) and/or the like. In addition, while particular examples are made of chlorhexidine base, chlorhexidine diacetate, and chlorhexidine dodecanoate, embodiments of the invention are not limited to any one form. Instead, as used herein, the term, 'chlorhexidine' refers to any one or a mixture of chlorhexidine base, pharmaceutically acceptable chlorhexidine salts such as, for example, diacetate, dodecanoate, palmitate, myristate, stearate and/or the like. For example, other suitable chlorhexidine salts are to be found in U.S. Patent 6,706,024, entitled Triclosan-Containing Medical Devices, issued on March 16, 2004. In general, suitable concentrations of chlorhexidine include a range from about 0.1% weight to weight (wt/wt) to about 30% wt/wt. More particularly, a suitable chlorhexidine range includes from about 3% wt/wt to about 20% wt/wt.

Suitable base materials generally include elastomers and/or polymer materials. Specific examples of suitable base materials include polyurethanes, polyvinylchlorides, thermoplastics such as, for example, fluoropolymers, vinyl polymers, polyolephins, copolymers, and/or the like.

In the following reference-embodiments (not according to the claimed invention) a duration of fibrinolytic activity is determined for urokinase-type plasminogen activator (uPA) immobilized on polyurethane catheter segments is determined. In addition, the unexpected improvements in the duration of fibrinolytic activity are determined for dextran sulfate stabilized uPA immobilized on polyurethane catheter segments is determined. We further show the unexpected improvements in the duration of fibrinolytic activity and antimicrobial activity when low molecular weight dextran sulfate is combined with uPA and an antimicrobial agent such as chlorhexidine and/or gentian violet.

### METHODS

### EXPERIMENT 1: Immobilizing an enzyme on polyurethane catheters, and determining the duration of enzymatic activity.

To immobilize an enzyme, polyurethane catheter segments were treated with 1% Butadiene Maleic Anhydride (BMA1:1 ratio) polymer (25% solution in acetone, Polysciences, Warrington PA) and 1% Polyethylene glycol 400 (PEG 400, Hampton Research, Aliso Viejo CA) in acetone followed by curing at 90-100°C for 3 hours under vacuum. Subsequently, the cured segments were incubated for 18 hours in sodium acetate buffer containing an enzyme, such as urokinase-type plasminogen activator (uPA), Streptokinase (SK), tissue plasminogen activator (tPA), collagenase, protease, aminopeptidase, matrix metalloproteinase, metalloendopeptidase, serine endopeptidase, a hydrolase, glucuronidase, hyaluronoglucosaminidase, heparanase, cathepsin, hyaluronidase, and matriptase, at 10 - 100 units/ µl depending on the type of enzyme used. Thereafter, the segments were rinsed with phosphate buffered saline twice and finally with deionized water.

The immobilization of collagenase, protease, aminopeptidase, matrix metalloproteinase, metalloendopeptidase, serine endopeptidase, hyaluronidase and matriptase are according to the claimed invention. The immobilization of urokinase-type plasminogen activator, streptokinase or tissue plasminogen activator represent reference-embodiments.

The experiments described herein below, all of which include urokinase-type plasminogen activator, are therefore to be considered reference-experiments.

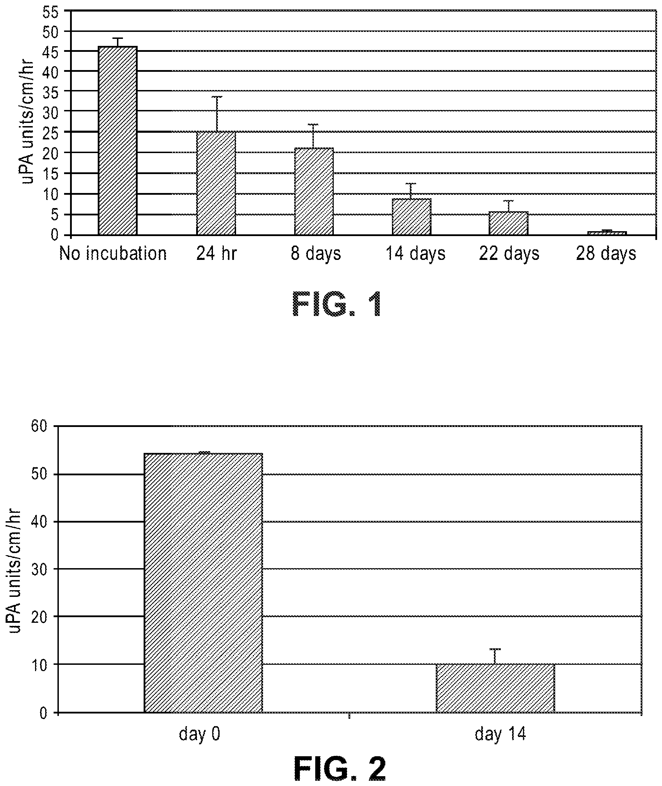

To test the durability of the activity from the immobilized enzyme, the catheters segments were incubated at 37°C in citrated human plasma for different time periods. The citrated human plasma was replaced with fresh plasma after every 7 days. The activity of the enzyme immobilized on a catheter surface was lost within 28 days, as shown for immobilized uPA in Figure 1.

### EXPERIMENT 2: Determining in vivo performance of a catheter with immobilized enzyme implanted in the superior vena cava of a rabbit

To assess *in vivo* performance of the catheters with immobilized uPA, single lumen size 6 French (Fr) catheters were implanted in the superior vena cava of rabbits for 14 days. Urokinase (uPA) activity was measured on the surface of explanted catheters via a chromogenic assay as is well known to those skilled in the art. As shown in Figure 2, the catheters retained about 20% of the original activity at day 14. The *in vivo* results correspond well with the amount of activity lost *in vitro* after fourteen days of incubation in human plasma (Figure 1).

### EXPERIMENT 3: Evaluating compounds for improving the performance of the catheters with enzymatic activity

Various compounds were evaluated for improving the duration of enzymatic activity from a catheter with immobilized enzyme. These compounds included: 1) low molecular weight dextran sulfate (LMW-DexS) with molecular weight of 8 kilo dalton (8 kDa); 2) human serum albumin (HSA); and 3) arginine. In this test, either 1 milligram/milliliter (mg/mL) of HSA or 1% weight/volume (w/v) of LMW-DexS (8 kDa) was added to a solution of sodium acetate buffer containing an enzyme at 10 - 100 units/µl. Either of the above solution was used to incubate BMA/PEG treated catheter segments. In addition, segments treated with BMA/PEG and the enzyme were rinsed in saline and, subsequently dipped in an aqueous solution containing 0.01% arginine as described in United States Patent No.: 4,764,466, entitled Method for stabilizing an immobilized fibrinolytic enzyme, issued on August 16, 1988.

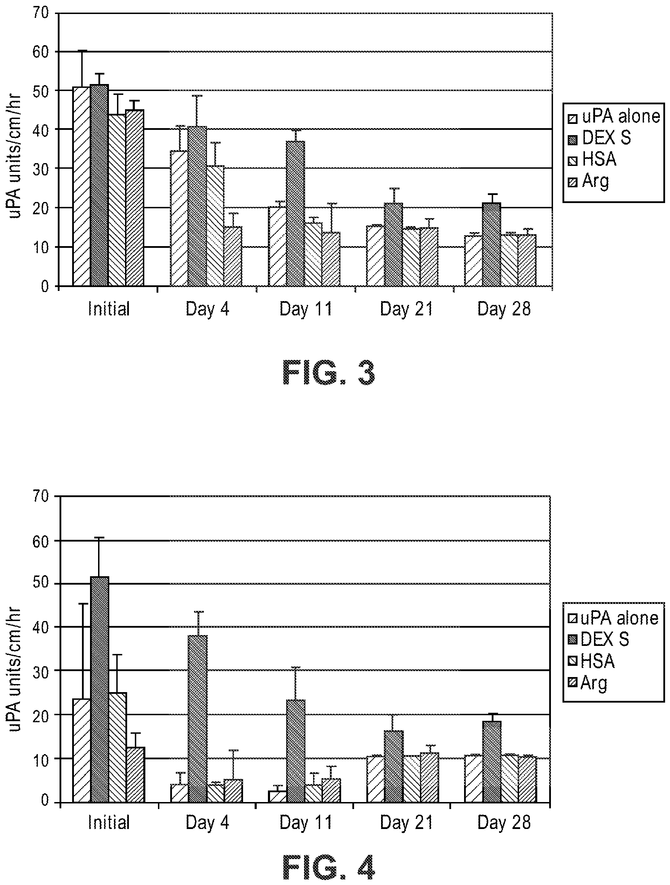

As shown in Figure 3, LMW-DexS (8 kDa) treated segments with immobilized uPA retained the highest enzyme activity after 28 days of incubation in water at 37°C compared to the other treatments.

### EXPERIMENT 4: Evaluating compounds for improved performance of the enzymatic catheters containing an antimicrobial agent

As disclosed in U.S. Patent Nos. 5,688,516, 6,273,875, and 6,528,107, medical devices may be treated with antimicrobial agents together with an enzyme to provide dual benefits. However, to date, no known research has been conducted to examine the use of LMW-DexS (8 kDa) to improve the bio-durability of implantable medical devices with antimicrobial agents together with an enzyme. To test if LMW-DexS (8 kDa) has similar stabilizing effect on an immobilized enzyme, such as uPA in presence of an antimicrobial agent, polyurethane catheters were dip coated in a polymer solution containing either chlorhexidine diacetate (CHA) or chlorhexidine dodecanoate (CHDD) and dried overnight at room temperature. The CHA or CHDD coated catheters were subsequently dipped in BMA/PEG solution for 30 seconds and then cured for 3 hours at 90-100°C under vacuum. Thereafter the cured segments were incubated in urokinase solution containing HSA, LMW-DexS (8 kDa) or arginine as in the earlier experiment.

As shown in Figure 4, of the three agents tested, LMW-DexS (8 kDa) treated segments maintained the highest uPA activity after 28 days of incubation in water.

### EXPERIMENT 5: Evaluating the use of maleic anhydride to attach an enzyme to the catheters containing an antimicrobial agent

Thin films rich in anhydride groups can be created by pulsed plasma polymerization of maleic anhydride. (See J Hu et al (2003) Functionalization of poly(ethylene terephthalate) film by pulsed plasma deposition of maleic anhydride. Advanced Functional Materials 13: 692- 697.) The plasma of maleic anhydride was utilized as a method to introduce anhydride groups for enzyme attachment on to the CHA/polyurethane surface. This method of enzyme attachment is generally known to those skilled in the art and thus, in the interest of brevity, is only briefly described herein. Polyurethane catheters were dip coated in a polymer solution containing CHA, dried overnight at room temperature, treated with maleic anhydride (Sigma-Aldrich Corp. St. Louis MO) plasma generated under Argon atmosphere in a plasma chamber (Diener Electronics, Reading PA). The plasma deposition reactions were carried out at a pressure of 15 Pascal (Pa), 90 Watts (W) peak power for 5 minutes or for ten cycles of 12 seconds each. Subsequently, catheter segments were incubated for 18 hours in sodium acetate buffer, pH 5 containing 30 units/µl uPA with or without 1% LMW-DexS (8 kDa). Catheter segments were rinsed in phosphate buffered saline (PBS) twice and then in deionized water before incubation in citrated human plasma for durability testing. After 28 days of incubation in human plasma, enzymatic (fibrinolytic) activity of catheter segments was assessed by a clot lysis method. The clots were made as follows: 9% fibrinogen and 1.7 units/mL of plasminogen were incubated at 37°C for 5 minutes in 197 millimolar (mM) borate/borax buffer, pH 7.5 containing 0.9% (w/v) sodium chloride and 0.5% (w/v) gelatin. Subsequently, 100units/mL of thrombin was added to initiate the fibrin polymerization and clot formation.

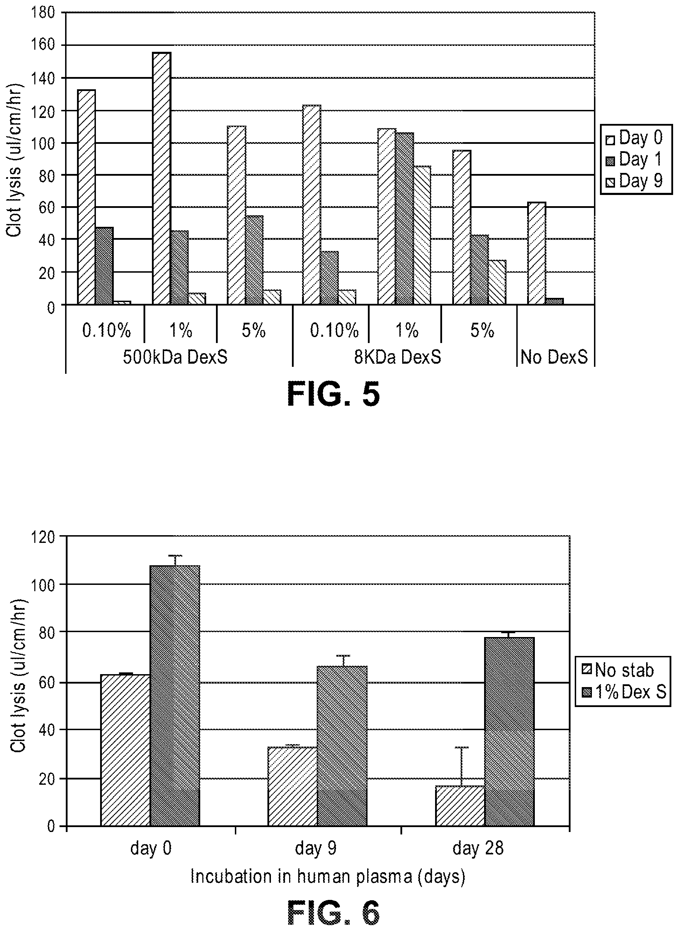

To conduct the clot lysis assay, a 0.5 cm long catheter segment was placed on a clot for 6 hours at 37°C and the volume of lysed clot was recorded. This procedure was performed for each sample shown in Figure 5. As shown in Figure 5, approximately an eight fold higher clot lysis was achieved when 1% LMW-DexS (8 kDa) was included as enzyme stabilizer compared to high molecular weight dextran sulfate or absence of dextran sulfate after 9 days of soaking in human plasma.

### EXPERIMENT 6: Evaluating the duration of enzymatic activity from the low molecular weight dextran sulfate stabilized catheter segments containing an antimicrobial agent

To evaluate the durability of the 1% LMW-DexS (8 kDa) treated catheters segments containing the antimicrobial agent, CHA, and immobilized enzyme, uPA, the catheter segments were incubated at 37°C in citrated human plasma for different time periods. The citrated human plasma was replaced with fresh sample after every 7 days. As shown in Figure 6, the 1% LMW-DexS (8 kDa) treated catheters segments retained approximately 80% of the enzymatic activity after 28 days. That is, the 1% LMW-DexS (8 kDa) stabilized and immobilized uPA enzyme remained active for 28 days in human plasma substantially without any loss in the activity compared to the control. These results were particularly unexpected in light of the approximately 90+% loss in activity shown in Figures 1 and 6 for un-stabilized uPA.

### EXPERIMENT 7: Evaluating antimicrobial activity for low molecular weight dextran sulfate stabilized catheter segments containing antimicrobial agents

In addition to fibrinolytic activity, antimicrobial activity of the 1% LMW-DexS (8 kDa) treated catheters was evaluated. In this test, adherence of gram positive bacteria *Staphylococcus aureus* ATCC 33591, on the catheter segments having immobilized enzyme uPA stabilized with 1% LMW-DexS (8 kDa), along with the antimicrobial agents such as gentian violet (0.6%) and chlorhexidine dodecanoate was compared to similarly treated catheter segments but without the LMW-DexS (8 kDa) stabilized enzyme. A five log reduction in adherence of the bacteria was observed on both catheter types, i.e. either with or without LMW-DexS (8 kDa). Thus, the antimicrobial activity of gentian violet and chlorhexidine dodecanoate against the tested organisms was not compromised due to the attachment of LMW-DexS (8 kDa) stabilized urokinase.

## Claims

1. A medical device, comprising:
a base material; and
an immobilized enzyme covalently bound to the base material in the presence of dextran sulfate, wherein the immobilized enzyme is an extracellular matrix digestive enzyme including one or more of:
a protease;
an aminopeptidase;
a collagenase;
a matrix metalloproteinase;
a metalloendopeptidase;
a serine endopeptidase;
a hydrolase;
a glucuronidase;
a hyaluronoglucosaminidase;
a heparanase;
a cathepsin;
a hyaluronidase; and
a matriptase;
wherein the medical device is configured to be implanted in the vascular system of a patient, and
wherein the dextran sulfate has a molecular weight of 8 kilodaltons (kDa) to stabilize the immobilized enzyme when in contact with blood of the patient.

2. The medical device according to claim 1, further comprising:
a combination of two or more antimicrobial agents impregnating the base material.

3. The medical device according to claim 2, wherein the combination of two or more antimicrobial agents includes:
a pharmaceutically acceptable salt of a bis-biguanide including at least one of:
a chlorhexidine base; and
an alexidine; and
a guanide including polyhexamethylene biguanide (PHMB).

4. The medical device according to claim 1, further comprising:
an antimicrobial agent impregnating the base material.

5. The medical device according to claim 4, wherein the antimicrobial agent includes:
a chlorhexidine base;
a pharmaceutically acceptable salt of a chlorhexidine base;
chlorhexidine diacetate;
alexidine; or
polyhexamethylene biguanide (PHMB).

6. The medical device of claim 1, wherein the base material is a polyurethane base material and wherein the amount of the extracellular matrix digestive enzyme is sufficient to reduce neo-initimal hyperplasia, and
wherein the device further comprises a blood clot digestive enzyme covalently bound to the base material in the presence of the dextran sulfate wherein the amount of the blood clot digestive enzyme is sufficient to reduce the formation of device related blood clots, and an antimicrobial agent disposed in the polyurethane base material in an amount sufficient to reduce microbial growth.

7. The medical device of claim 1, wherein the base material is a polyurethane base material and wherein the amount of the extracellular matrix digestive enzyme is sufficient to reduce neo-initimal hyperplasia, and wherein the device further comprises a chlorhexidine base or a pharmaceutically acceptable salt thereof disposed in the polyurethane base material in an amount sufficient to reduce microbial growth.

8. The medical device of claim 1, further comprising a cationic selective antimicrobial impregnating the base material.

9. The medical device according to claim 1 or 8, wherein at least a part of the medical device has a tubular structure including the base material.

10. The medical device according to claim 1 or 8, wherein the base material is a polymer.

11. The medical device according to claim 10, wherein the polymer is a polyurethane.

12. The medical device according to claim 8, wherein the cationic selective antimicrobial is a ceragenin.

13. The medical device according to claim 8, wherein the cationic selective antimicrobial is in an amount sufficient to reduce microbial growth.

## Patentansprüche

1. Medizinische Vorrichtung, umfassend:
ein Basismaterial; und
ein immobilisiertes Enzym, das kovalent an das Basismaterial in der Gegenwart von Dextransulfat gebunden ist, wobei das immobilisierte Enzym ein extrazelluläres Matrix-Verdauungsenzym ist, einschließlich:
einer Protease;
einer Aminopeptidase;
einer Kollagenase;
einer Matrix-Metalloproteinase;
einer Metalloendopeptidase;
einer Serin-Endopeptidase;
einer Hydrolase;
einer Glucuronidase;
einer Hyaluronoglukosaminidase;
einer Heparanase;
eines Kathepsins;
einer Hyaluronidase; und/oder
einer Matriptase;
wobei die medizinische Vorrichtung konfiguriert ist, um in das Gefäßsystem eines Patienten implantiert zu werden, und wobei das Dextransulfat ein Molekulargewicht von 8 Kilodalton (kDa) aufweist, um das immobilisierte Enzym bei Kontakt mit Blut des Patienten zu stabilisieren.

2. Medizinische Vorrichtung nach Anspruch 1, ferner umfassend:
eine Kombination aus zwei oder mehr antimikrobiellen Mitteln, die das Basismaterial imprägnieren.

3. Medizinische Vorrichtung nach Anspruch 2, wobei die Kombination aus zwei oder mehr antimikrobiellen Mitteln Folgendes einschließt:
ein pharmazeutisch unbedenkliches Salz eines Bis-Biguanids, einschließlich:
einer Chlorhexidinbase; und
eines Alexidins; und/oder
eines Guanids, einschließlich Polyhexamethylenbiguanid (PHMB).

4. Medizinische Vorrichtung nach Anspruch 1, ferner umfassend:
ein antimikrobielles Mittel, das das Basismaterial imprägniert.

5. Medizinische Vorrichtung nach Anspruch 4, wobei das antimikrobielle Mittel Folgendes einschließt:
eine Chlorhexidinbase;
ein pharmazeutisch unbedenkliches Salz einer Chlorhexidinbase;
Chlorhexidindiazetat;
Alexidin; oder
Polyhexamethylenbiguanid (PHMB).

6. Medizinische Vorrichtung nach Anspruch 1, wobei das Basismaterial ein Polyurethan-Basismaterial ist und wobei die Menge des extrazellulären Matrix-Verdauungsenzyms ausreichend ist, um neoinitimale Hyperplasie zu reduzieren, und wobei die Vorrichtung ferner ein Blutgerinnsel-Verdauungsenzym umfasst, das kovalent an das Basismaterial in der Gegenwart des Dextransulfats gebunden ist, wobei die Menge des Blutgerinnsel-Verdauungsenzyms ausreichend ist, um das Ausbilden von vorrichtungsbezogenen Blutgerinnseln zu reduzieren, und ein antimikrobielles Mittel, das in dem Polyurethan-Basismaterial in einer Menge angeordnet ist, die ausreicht, um mikrobielles Wachstum zu reduzieren.

7. Medizinische Vorrichtung nach Anspruch 1, wobei das Basismaterial ein Polyurethan-Basismaterial ist und wobei die Menge des extrazellulären Matrix-Verdauungsenzyms ausreichend ist, um neoinitimale Hyperplasie zu reduzieren, und wobei die Vorrichtung ferner eine Chlorhexidinbase oder ein pharmazeutisch unbedenkliches Salz davon umfasst, das in dem Polyurethan-Basismaterial in einer Menge angeordnet ist, die ausreichend ist, um mikrobielles Wachstum zu reduzieren.

8. Medizinische Vorrichtung nach Anspruch 1, ferner umfassend ein kationisches selektives antimikrobielles Mittel, das das Basismaterial imprägniert.

9. Medizinische Vorrichtung nach Anspruch 1 oder 8, wobei wenigstens ein Teil der medizinischen Vorrichtung eine röhrenförmige Struktur aufweist, die das Basismaterial einschließt.

10. Medizinische Vorrichtung nach Anspruch 1 oder 8, wobei das Basismaterial ein Polymer ist.

11. Medizinische Vorrichtung nach Anspruch 10, wobei das Polymer ein Polyurethan ist.

12. Medizinische Vorrichtung nach Anspruch 8, wobei das kationische selektive antimikrobielle Mittel ein Ceragenin ist.

13. Medizinische Vorrichtung nach Anspruch 8, wobei das kationische selektive antimikrobielle Mittel in einer Menge vorliegt, die ausreichend ist, um mikrobielles Wachstum zu reduzieren.

## Revendications

1. Dispositif médical comprenant :
un matériau de base ; et
une enzyme immobilisée liée de manière covalente au matériau de base en présence de sulfate de dextran, l'enzyme immobilisée étant une enzyme digestive de matrice extracellulaire comprenant un ou plusieurs parmi les éléments suivants :
une protéase ;
une aminopeptidase ;
une collagénase ;
une métalloprotéinase matricielle ;
une métalloendopeptidase ;
une sérine endopeptidase ;
une hydrolase ;
une glucuronidase ;
une hyaluronoglucosaminidase ;
une héparanase ;
une cathepsine ;
une hyaluronidase ; et
une matriptase ;
le dispositif médical étant configuré pour être implanté dans le système vasculaire d'un patient et le sulfate de dextran ayant un poids moléculaire de 8 kilodaltons (kDa) pour stabiliser l'enzyme immobilisée lorsqu'elle est en contact avec le sang du patient.

2. Dispositif médical selon la revendication 1, comprenant en outre :
une combinaison d'au moins deux agents antimicrobiens imprégnant le matériau de base.

3. Dispositif médical selon la revendication 2, dans lequel la combinaison d'au moins deux agents antimicrobiens comprend :
un sel pharmaceutiquement acceptable d'un bis-biguanide comprenant au moins l'un des éléments suivants :
une base de chlorhexidine ;
une alexidine ; et
un guanide comprenant du polyhexaméthylène biguanide (PHMB).

4. Dispositif médical selon la revendication 1, comprenant en outre :
un agent antimicrobien imprégnant le matériau de base.

5. Dispositif médical selon la revendication 4, dans lequel l'agent antimicrobien comprend :
une base de chlorhexidine ;
un sel pharmaceutiquement acceptable d'une base de chlorhexidine ;
le diacétate de chlorhexidine ;
l'alexidine ; ou
le polyhexaméthylène biguanide (PHMB).

6. Dispositif médical selon la revendication 1, dans lequel le matériau de base est un matériau à base de polyuréthane et dans lequel la quantité de l'enzyme digestive de matrice extracellulaire est suffisante pour réduire l'hyperplasie néo-initimale, et dans lequel le dispositif comprend en outre une enzyme digestive de caillot sanguin liée de manière covalente au matériau de base en présence du sulfate de dextran, la quantité de l'enzyme digestive de caillot sanguin étant suffisante pour réduire la formation de caillots sanguins liés au dispositif, et un agent antimicrobien étant disposé dans le matériau à base de polyuréthane en une quantité suffisante pour réduire la croissance microbienne.

7. Dispositif médical selon la revendication 1, dans lequel le matériau de base est un matériau à base de polyuréthane, dans lequel la quantité de l'enzyme digestive de matrice extracellulaire est suffisante pour réduire l'hyperplasie néo-initimale, et dans lequel le dispositif comprend en outre une base de chlorhexidine ou un de ses sels pharmaceutiquement acceptables disposé dans le matériau à base de polyuréthane en une quantité suffisante pour réduire la croissance microbienne.

8. Dispositif médical selon la revendication 1, comprenant en outre un antimicrobien à sélectivité cationique imprégnant le matériau de base.

9. Dispositif médical selon la revendication 1 ou 8, dans lequel au moins une partie du dispositif médical présente une structure tubulaire comprenant le matériau de base.

10. Dispositif médical selon la revendication 1 ou 8, dans lequel le matériau de base est un polymère.

11. Dispositif médical selon la revendication 10, dans lequel le polymère est un polyuréthane.

12. Dispositif médical selon la revendication 8, dans lequel l'antimicrobien à sélectivité cationique est un composé céragénine.

13. Dispositif médical selon la revendication 8, dans lequel l'antimicrobien à sélectivité cationique est présent en une quantité suffisante pour réduire la croissance microbienne.
